(19) Europäisches Patentamt
European Patent Office
Office européen des brevets

(11) Veröffentlichungsnummer : **0 402 744 B1**

(12) # EUROPÄISCHE PATENTSCHRIFT

(45) Veröffentlichungstag der Patentschrift :
**31.03.93 Patentblatt 93/13**

(51) Int. Cl.$^5$ : **C07C 29/86,** C07C 33/035, C07C 29/56

(21) Anmeldenummer : **90110651.8**

(22) Anmeldetag : **06.06.90**

(54) **Verfahren zur Gewinnung von trans-1,1,4,4-tetraalkyl-2-buten-1,4-diolen.**

(30) Priorität : **13.06.89 DE 3919226**

(43) Veröffentlichungstag der Anmeldung :
**19.12.90 Patentblatt 90/51**

(45) Bekanntmachung des Hinweises auf die Patenterteilung :
**31.03.93 Patentblatt 93/13**

(84) Benannte Vertragsstaaten :
**BE DE FR GB IT NL**

(56) Entgegenhaltungen :
**DE-A- 3 417 943**
**US-A- 3 352 929**
**US-A- 4 596 643**
**CHEMICAL ABSTRACTS, Band 82, Nr. 25, 23. Juni 1975, S. 476, Zusammenfassung Nr. 170211e, Columbus, Ohio, USA; & JP-A-49 100 055**

(56) Entgegenhaltungen :
**BEILSTEINS HANDBUCH DER ORGANISCHEN CHEMIE, 4. Auflage, Band 1, Julius-Springer-Verlag, Berlin, DE; I. Ergänzungswerk (1928), III: Ergänzungswerk (1958)**

(73) Patentinhaber : **BASF Aktiengesellschaft**
**Carl-Bosch-Strasse 38**
**W-6700 Ludwigshafen (DE)**

(72) Erfinder : **Bender, Dietmar, Dr.**
**Sebastian-Kneipp-Strasse 19**
**W-6707 Schifferstadt (DE)**
Erfinder : **Bronstert, Klaus, Dr.**
**Gartenstrasse 26**
**W-6719 Carlsberg (DE)**
Erfinder : **Fischer, Martin, Dr.**
**Elbinger Weg 1**
**W-6700 Ludwigshafen (DE)**
Erfinder : **Schuermann, Gregor, Dr.**
**Werderstrasse 40**
**W-6900 Heidelberg (DE)**

EP 0 402 744 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft ein Verfahren zur Gewinnung von trans-1,1,4,4-Tetraalkyl-2-buten-1,4-diolen der Formel I

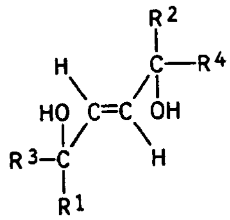

I

in der die Reste $R^1$, $R^2$, $R^3$ und $R^4$ gleich oder verschieden sind und für $C_1$- bis $C_5$-Alkylgruppen stehen, aus Gemischen mit ihrem cis-Isomeren.

Die reinen trans-Diole der Formel I und besonders bevorzugt die trans-Diole der Formel II dienen als Ausgangsmaterialien zur Synthese von Initiator-Transfer-Reagenzien, sogenannter "Inifer-Initiatoren", das sind Initiatoren für die kationische Polymerisation (s. dazu: EP-A 0265053; EP-A 0206756; Polymer Bull. 19, 427 (1988); Polymer Bull. 18, 433 (1987)).

Einer breiten Anwendung solcher Initiatoren stand bislang die aufwendige Synthese der reinen trans-Diole I und insbesondere der reinen trans-2,5-Dialkyl-3-hexen-2,5-diole II entgegen. Diese sind auf synthetischem Wege nur über die Reduktion von 2,5-Dimethyl-3-hexin-2,5-diol mit Lithiumaluminiumhydrid (Liebigs Ann. Chem. 608, 195 (1957)) oder die Reduktion von polymeren Peroxiden des 2,5-Dimethyl-2,4hexadiens mit Hilfe von Thiolen (J.Org. Chem. 29, 1887 (1964)) zugänglich - beide Verfahren sind jedoch im industriellen Maßstab impraktikabel und unwirtschaftlich.

Im Gegensatz zu den reinen trans-Verbindungen I können Gemische aus cis- und trans-1, 1,4,4-Tetraalkyl-2-buten-1,4-diolen auf wirtschaftliche Weise hergestellt werden, beispielsweise durch photochemische oder katalytische Isomerisierung des jeweiligen cis-Isomeren (Helv. Chim. Acta 51, 548 (1968); DE-A 3417943) oder durch die radikalische Addition von sek. Alkoholen an 2-Alkyl-3-butin-2-ole (US-A 3304247; US-A 3352929). Die zur photochemischen Isomerisierung benötigten cis-Isomeren sind durch die katalytische Reduktion von trans-1,1,4,4-Tetraalkyl-2-butin-1,4-diolen mit Wasserstoff in sehr guten Ausbeuten erhältlich (vgl. J. Org. Chem. 29, 1887 (1964)). Infolge der großen strukturellen Ähnlichkeit von cis- und trans-Verbindungen und ihres sehr ähnlichen physikalischen Verhaltens war aber bisher kein Verfahren bekannt, das es erlaubt, trans-1,1,4,4-Tetraalkyl-2-buten-1,4-diole und insbesondere trans-2,5-Dialkyl-3-hexen-2,5-diole im industriellen Maßstab wirtschaftlich aus ihren Gemischen mit dem cis-Isomeren zu gewinnen. Es war deshalb die Aufgabe, ein solches Verfahren zu finden.

Dementsprechend wurde ein Verfahren zur Gewinnung von trans-1,1,4,4-Tetralkyl-2-buten-1,4-diolen der Formel I

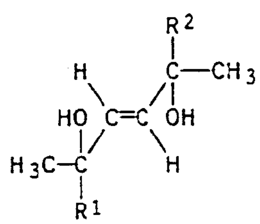

I

in der die Reste $R^1$, $R^2$, $R^3$ und $R^4$ gleich oder verschieden sind und für $C_1$- bis $C_5$-Alkylgruppen stehen, aus Gemischen mit ihrem cis-Isomeren gefunden, das dadurch gekennzeichnet ist, daß man das trans-Isomere mittels Flüssig-Flüssig-Extraktion mit einem Zwei-Phasen-Gemisch, bestehend aus einer polaren und einer unpolaren Phase, vom cis-Isomeren abtrennt.

Das erfindungsgemäße Verfahren eignet sich zur Gewinnung der trans-Alkendiole der Formel I, in der die Reste $R^1$, $R^2$, $R^3$ und $R^4$ gleich oder verschieden sein können und für eine $C_1$- bis $C_5$-Alkylgruppe stehen können, aus ihren Gemischen mit dem cis-Isomeren. Bevorzugt wird das erfindungsgemäße Verfahren zur Gewinnung von trans-Alkendiolen I, in denen die Reste $R^3$ und $R^4$ einer Methylgruppe entsprechen, also zur Gewinnung von trans-2,5-Dialkyl-3-hexen-2,5-diolen der allgemeinen Formel II

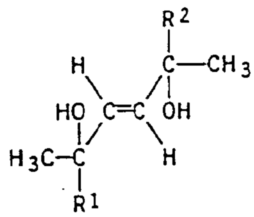

II

verwendet.

Besonders bevorzugt dient das erfindungsgemäße Verfahren zur Gewinnung von trans-Alkendiolen II, in denen die Reste $R^1$ und $R^2$ gleich sind und besonders vorteilhaft zur Gewinnung von trans-2,5-Dimethyl-3-hexen-2,5-diol.

Das erfindungsgemäße Flüssig-Flüssig-Extraktionsverfahren kann kontinuierlich oder im chargenweisen Betrieb in handelsüblichen Flüssig-Flüssig-Extraktionsapparaten wie sie beispielsweise in Ullmanns Encyklopädie der techn. Chemie, Bd. 2, S. 560-565, 4. Auflage, Verlag Chemie, Weinheim, 1972, beschrieben sind, ausgeübt werden. Zur Extraktion wird ein Zwei-Phasen-Gemisch, bestehend aus einer polaren und einer unpolaren Phase verwendet, wobei sich das cis- und das trans-Isomere überraschenderweise in den beiden Phasen derart unterschiedlich stark anreichern, daß eine selektive Abtrennung des trans-Isomeren möglich wird.

Als polare Phase können Wasser oder Gemische von Wasser mit anderen polaren, gut wasserlöslichen Lösungsmitteln, wie cyclischen Harnstoffen, Dimethylformamid, Tetrahydrofuran, Methoxyethanol, Dimethylsulfoxid, Hexamethylphosphorsäuretriamid, Aceton oder niederen Alkoholen, insbesondere Methanol und Ethanol, verwendet werden. Bei der Verwendung von Wasser-Lösungsmittel-Gemischen als polare Phase ist jedoch darauf zu achten und sollte ggf. durch einen Vorversuch abgesichert werden, daß diese Gemische keine Emulsionen mit der unpolaren Phase bilden. Bevorzugt werden Wasser oder Mischungen von Wasser mit $C_1$- bis $C_4$-Alkoholen, insbesondere mit Methanol und/oder Ethanol als polare Phase eingesetzt. Die Nutzung von Lösungen höherer Alkohole in Wasser als polare Phase ist ebenfalls möglich und der Verwendung der $C_1$- bis $C_4$-Alkohol/Wasser-Mischungen äquivalent. Allerdings führt die Verwendung solcher höherer Alkohole, aufgrund ihrer mäßigen Wasserlöslichkeit, zu keinen weiteren Vorteilen, verglichen mit der Verwendung niederer Alkohole.

Als unpolare Phase dienen bevorzugt aliphatische, alkylaromatische oder aromatische Kohlenwasserstoffe sowie Mischungen dieser Lösungsmittel. Gute Resultate können außerdem bei Verwendung von halogenierten aliphatischen oder aromatischen Kohlenwasserstoffen oder von langkettigen (Zahl der Kohlenstoffatome größer als 5) Ketonen allein oder in Mischungen mit den genannten unpolaren Lösungsmitteln als unpolare Phase erhalten werden.

Es versteht sich von selbst, daß sich die als Extraktionsmittel verwendeten Lösungsmittel gegenüber dem Alkendiol chemisch inert verhalten sollen. Damit eine Phasentrennung stattfinden kann, ist es weiterhin erforderlich, daß sich polare und unpolare Phase in ihrer Dichte unterscheiden: je größer der Dichteunterschied dieser Phasen ist, desto schneller geht die Phasentrennung vonstatten.

Bei der Wahl der Lösungsmittel für die polare und die unpolare Phase sollte zur Erzielung einer möglichst vollständigen, möglichst effizienten und wirtschaftlichen Trennung der cis- und trans- isomeren-Alkendiole I darauf geachtet werden, daß der Quotient der Verteilungskoeffizienten $k_{trans}/k_{cis}$ des trans- und cis-Isomeren zwischen der polaren und unpolaren Phase möglichst groß ist. Der Verteilungskoeffizient k des cis- oder trans-Isomeren ist dabei definiert als der Quotient aus der Konzentration des betreffenden Isomeren in der polaren Phase und seiner Konzentration in der unpolaren Phase nach Einstellung des Verteilungsgleichgewichts, gemäß den Gleichungen (1) und (2):

$$(1) \qquad k_{cis} = \frac{\text{Konzentration des cis} - \text{Isomeren in der polaren Phase}}{\text{Konzentration des cis} - \text{Isomeren in der unpolaren Phase}}$$

$$(2) \qquad k_{cis} = \frac{\text{Konzentration des trans} - \text{Isomeren in der polaren Phase}}{\text{Konzentration des trans} - \text{Isomeren in der unpolaren Phase}}$$

Tabelle 1 (s. Beispiele) gibt beispielhaft die für cis- und trans-2,5-Dimethyl-3-hexen-2,5-diol ermittelten Verteilungskoeffizienten k zwischen verschiedenerlei polaren und unpolaren Phasen wieder. Aus den darin aufgeführten Verteilungskoeffizienten ergibt sich, daß sich das cis-Isomere des 2,5-Dimethyl-3-hexen-2,5-diol in der unpolaren, sein trans-Isomeres sich hingegen in der polaren Phase anreichert. Alle in Tabelle 1 genannten Lösungsmittel und Lösungsmittelgemische sind für das erfindungsgemäße Verfahren geeignet.

Das erfindungsgemäße Extraktionsverfahren wird im allgemeinen bei Atmosphärendruck oder unter dem Eigendruck der Extraktionsmittel und bei Temperaturen zwischen 0 und 100°C, vorzugsweise bei 15 bis 85°C,

ausgeführt. Die Extraktionstemperatur wird im allgemeinen so gewählt, daß sie sich unterhalb des Siedepunktes eines der verwendeten Lösungsmittel befindet. Liegt die gewählte Extraktionstemperatur oberhalb des Siedepunktes eines der verwendeten Extraktionsmittel, so ist es oftmals vorteilhaft, die Extraktion unter dem Eigendruck der Extraktionsmittel oder unter exogen erhöhtem Druck durchzuführen.

Zweckmäßigerweise sollten die Extraktionsmittel in solchen Mengen bezüglich des aufzutrennenden cis-trans-Alkendiol-Gemisches verwendet werden, daß das trans- bzw. das cis-Isomere von der polaren bzw. der unpolaren Phase vollständig gelöst werden kann. Größere Mengen an Extraktionsmittel können sich vorteilhaft auswirken, doch ist dabei zu berücksichtigen, daß die Extraktionsmittel zur Isolierung des cis- und des trans-Isomeren wieder abdestilliert werden müssen.

Das Volumenverhältnis polare/unpolare Phase kann gleichfalls in weiten Grenzen variiert werden, im allgemeinen liegt dieses Verhältnis im Bereich von 0,1 bis 10, vorzugsweise im Bereich von 0,8 bis 3.

In der Praxis geht man im allgemeinen so vor, daß man die cis- trans-Isomerengemische, wie sie beispielsweise bei der Addition von sekundären Alkoholen an 2-Alkyl-3-butin-2-ole erhalten werden, im zweiphasigen Extraktionsmittelgemisch auflöst. Bei diesem Lösevorgang geht bereits ein großer Teil des cis-Alkendiols in die unpolare Phase über. Diese kann abgetrennt werden. Die polare Phase, welche noch beide Isomere enthält, kann dann anschließend, beispielsweise in einem Gegenstromextraktor, mit dem Lösungsmittel der unpolaren Phase extrahiert werden. Nach dieser Behandlung kann das trans-Isomere nach Abdampfen der polaren Phase, gegebenenfalls nach Abdampfen unter vermindertem Druck, als Eindampfrückstand praktisch isomerenrein gewonnen werden. Zweckmäßigerweise werden die vereinigten unpolaren Phasen, welche das cis-Isomer angereichert enthalten, nochmals mit dem Lösungsmittel der polaren Phase extrahiert, so daß sie am Ende nur noch geringe Mengen der trans-Verbindung enthalten. Das in der unpolaren Phase nun fast ausschließlich vorliegende cis-Alkendiol wird dann, vorzugsweise photochemisch, isomerisiert und die nun das cis-trans-Isomerengemisch enthaltende, unpolare Phase entweder in die erste Extraktionsstufe zurückgeführt oder vor der Rückführung einmal oder mehrere Male, vorzugsweise einmal, mit dem Lösungsmittel der polaren Phase extrahiert. Die photochemische Isomerisierung des cis-Alkendiols kann nach an sich bekannten Verfahren erfolgen, beispielsweise gemäß dem Verfahren der DE-A 3417943. Auf diese Weise läßt sich das cis-trans-Isomerengemisch der 2,5-Dialkyl-3-hexen-2,5-diole nach und nach praktisch quantitativ in das reine trans-Isomere überführen und als solches in guten Ausbeuten isolieren.

## Beispiele

2,5-Dimethyl-3-hexen-2,5-diol

2,5-Dimethyl-3-hexen-2,5-diol wurde durch radikalische Addition von Isopropanol an 2-Methyl-3-butin-2-ol nach dem Verfahren von US-A 3352929 hergestellt.

Ermittlung der Verteilungskoeffizienten für cis- und trans-2,5-Dimethyl-3-hexen-2,5-diol.

2 g cis- bzw. trans-Alkendiol wurden bei 25°C in einer Mischung aus 40 ml eines polaren Lösungsmittels und 40 ml eines unpolaren Lösungsmittels gelöst. Die Mischung wurde in einem Scheidetrichter 10 Minuten lang intensiv geschüttelt. Nach Trennung der beiden Phasen wurden die Lösungsmittel bei vermindertem Druck abdestilliert. Die Rückstände wurden gewogen und aus dieser Messung die Verteilungskoeffizienten nach den Gleichungen (1) und (2) bestimmt (vgl. Tabelle 1).

Tabelle 1:
Verteilungskoeffizienten von cis- und trans-2,5-Dimethyl-3-hexen-2,5-diol

| 2,5-Diol | polare Phase | unpolare Phase | k |
|---|---|---|---|
| cis | Wasser | Methylcyclohexan | 0.61 |
| cis | Wasser | Cyclohexan | 0.56 |
| cis | Wasser | n-Hexan | 0.76 |
| cis | Wasser | Toluol | 0.38 |
| cis | Wasser | Methylenchlorid | 0.17 |
| cis | Wasser | Methylisobutylketon | 0.09 |
| cis | Wasser/ 2 % Methanol | Toluol | 0.39 |
| cis | Wasser/ 10 % Ethanol | Toluol | 0.35 |
| trans | Wasser | Methylcyclohexan | 52 |
| trans | Wasser | Cyclohexan | 30 |
| trans | Wasser | n-Hexan | 34 |
| trans | Wasser | Toluol | 25 |
| trans | Wasser | Methylenchlorid | 7.3 |
| trans | Wasser | Methylisobutylketon | 3.0 |
| trans | Wasser/ 2 % Methanol | Toluol | 62 |
| trans | Wasser/ 10 % Ethanol | Toluol | 23 |

Photochemische Isomerisierung von cis-2,5-Dimethyl-3-hexen-2,5-diol

In einer 200 ml fassenden Belichtungsapparatur wurden 120 g cis-2,5-Dimethyl-3-hexen-2,5-diol mit 1,2 g Diphenylsulfid versetzt. Diese Mischung wurde mit Hilfe einer 300 W Hg-Hochdrucklampe 48 Stunden lang bei einer Temperatur von 90°C bestrahlt. Nach 10 h hatte sich ein cis/trans-Verhältnis von 46/54, nach 48 h ein cis/trans-Verhältnis von 22/78 eingestellt. Die cis/trans-Verhältnisse wurden NMR-spektroskopisch und gaschromatographisch bestimmt.

Gewinnung von trans-2,5-Dimethyl-3-hexen-2,5-diol aus seinem cis/trans-Isomerengemisch

25 kg eines Gemisches aus 30 % cis- und 70 % trans-2,5-Dimethyl-3-hexen-2,5-diol wurden in einer Mischung aus 100 l Wasser und 10 l Toluol bei 50°C gelöst. Die organische Phase, in der sich bereits beim Lösungsvorgang die Hälfte des cis-Alkendiols angereichert hatte, wurde abgetrennt. In einem Flüssig-Flüssig-Gegenstromextraktor mit einer Siebbodenkolonne (30 Siebböden; Kolonnendurchmesser: 50 cm; Füllhöhe der Kolonne: 100 cm) und Pulsationspumpe wurde die wäßrige Phase bei 50°C mit Toluol extrahiert (Durchsatz: 3 l/h wäßrige Lösung - 2 l/h Toluol). Nach dem Eindampfen der wäßrigen Phase wurden 8,5 kg trans-2,5-Dimethyl-3-hexen-2,5-diol in einer Reinheit von 99 % erhalten. Die vereinigten Toluol-Phasen wurden unter den oben genannten Bedingungen nochmals mit 100 l Wasser extrahiert. Während in der Toluol-Phase eine Mischung aus 95 % cis- und 5 % trans-Isomerem zurückblieb, reicherte sich das trans-Isomere in der wäßrigen Phase auf ca. 85 % an. Die wäßrige Phase wurde nochmals mit Toluol extrahiert und anschließend eingedampft. Als Eindampfrückstand wurden nochmals 7 kg des trans-Isomeren in einer Reinheit von 98,5 % erhalten. Bezogen auf die Einwaage betrug die Ausbeute an reinem trans-Isomeren über 88 %. Das in der organischen Phase angereicherte cis-Isomere wurde nach dem Entfernen des Lösungsmittels photochemisch teilweise in das trans-Isomere umgewandelt und das so erhaltene cis-trans-Isomerengemisch wieder in den Extraktionszyklus zurückgeführt.

## Patentansprüche

1. Verfahren zur Gewinnung von trans-1,1,4,4-Tetraalkyl-2-buten-1,4-diolen der Formel I

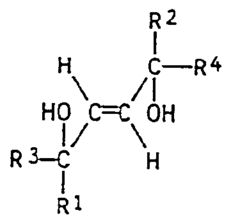

I

in der die Reste $R^1$, $R^2$, $R^3$ und $R^4$ gleich oder verschieden sind und für $C_1$- bis $C_5$-Alkylgruppen stehen, aus Gemischen mit ihrem cis-Isomeren, dadurch gekennzeichnet, daß man das trans-Isomere mittels Flüssig-Flüssig-Extraktion mit einem Zwei-Phasen-Gemisch, bestehend aus einer polaren und einer unpolaren Phase, vom cis-Isomeren abtrennt.

2. Verfahren nach Anspruch 1, dadurch gekennzeichnet, daß man trans-2,5-Dialkyl-3-hexen-2,5-diole der Formel II

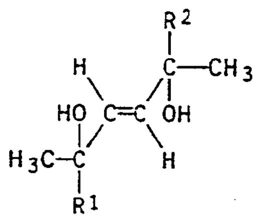

II

mittels Flüssig-Flüssig-Extraktion aus ihren Gemischen mit dem jeweiligen cis-Isomeren isoliert.

3. Verfahren nach Anspruch 1, dadurch gekennzeichnet, daß man das cis-Isomere mit der unpolaren Phase abtrennt.

4. Verfahren nach Anspruch 1, dadurch gekennzeichnet, daß man als polare Phase Wasser oder Mischungen von Wasser mit $C_1$- bis $C_4$-Alkoholen verwendet.

5. Verfahren nach Anspruch 1, dadurch gekennzeichnet, daß man als polare Phase Wasser oder Mischungen von Wasser mit Methanol und/oder Ethanol verwendet.

6. Verfahren gemäß Anspruch 1, dadurch gekennzeichnet, daß man das trans-Isomere mittels Flüssig-Flüssig-Extraktion vom cis-Isomeren abtrennt, das cis-Isomere anschließend zu einem Gemisch aus cis- und trans-Isomeren isomerisiert und aus diesem Gemisch wiederum das trans-Isomere durch Flüssig-Flüssig-Extraktion abtrennt.

7. Verfahren nach Anspruch 6, dadurch gekennzeichnet, daß man die Isomerisierung photochemisch durchführt.

## Claims

1. A process for obtaining trans-1,1,4,4-tetraalkyl-2-butene-1,4-diols of the formula I

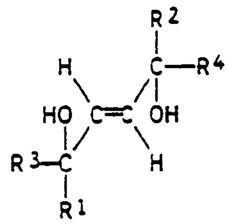

I

where $R^1$, $R^2$, $R^3$ and $R^4$ are identical or different, and are $C_1$-$C_5$-alkyl groups, from mixtures with their cisisomer, which comprises separating the transisomer from the cisisomer by means of liquid-liquid extraction with a two-phase mixture composed of a polar and of a non-polar phase.

2. A process as claimed in claim 1, wherein trans-2,5-dialkyl-3-hexene-2,5-diols of the formula II

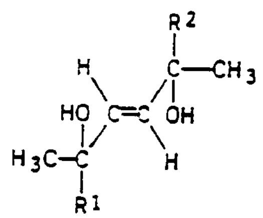

II

are isolated from their mixtures with the relevant cisisomer by means of liquid-liquid extraction.

3. A process as claimed in claim 1, wherein the cisisomer is removed with the non-polar phase.

4. A process as claimed in claim 1, wherein water or mixtures of water with $C_1$- to $C_4$-alcohols are used as polar phase.

5. A process as claimed in claim 1, wherein water or mixtures of water with methanol and/or ethanol are used as polar phase.

6. A process as claimed in claim 1, wherein the trans-isomer is separated from the cisisomer by means of liquid-liquid extraction, the cisisomer is subsequently isomerized to a mixture of cis and transisomers, and the transisomer is in turn removed from this mixture by liquid-liquid extraction.

7. A process as claimed in claim 6, wherein the isomerization is carried out photochemically.

## Revendications

1. Procédé pour la récupération de trans-1,1,4,4-tétraalkyl-2-butène-1,4-diols de formule I

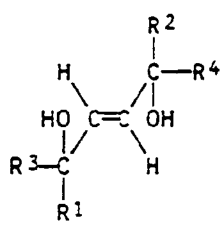

I

dans laquelle les restes $R^1$, $R^2$, $R^3$ et $R^4$ sont identiques ou différents et sont mis pour des groupements alkyle en $C_1$ à $C_5$, à partir de mélanges avec leurs isomères cis, caractérisé en ce qu'on sépare l'isomère trans de l'isomère cis par extraction liquide-liquide avec un mélange à deux phases se composant d'une phase polaire et d'une phase non polaire.

2. Procédé selon la revendication 1, caractérisé en ce qu'on isole des trans-2,5-dialkyl-3-hexène-2,5-diols de formule II

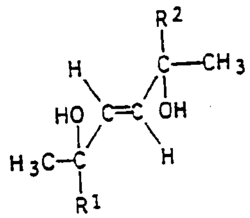

II

de leurs mélanges avec l'isomère cis respectif par extraction liquide-liquide.

3. Procédé selon la revendication 1, caractérisé en ce qu'on sépare l'isomère cis avec la phase non polaire.

4. Procédé selon la revendication 1, caractérisé en ce qu'on utilise, comme phase polaire, de l'eau ou des mélanges d'eau et d'alcools en $C_1$ à $C_4$.

5. Procédé selon la revendication 1, caractérisé en ce qu'on utilise, comme phase polaire, de l'eau ou des mélanges d'eau et de méthanol et/ou d'éthanol.

6. Procédé selon la revendication 1, caractérisé en ce qu'on sépare l'isomère trans de l'isomère cis par extraction liquide-liquide, puis on isomérise l'isomère cis en un mélange d'isomères cis et trans et on sépare de nouveau l'isomère trans de ce mélange par extraction liquide-liquide.

7. Procédé selon la revendication 6, caractérisé en ce qu'on effectue l'isomérisation par voie photochimique.